# EUROPEAN PATENT SPECIFICATION

(11) **EP 2 530 468 B1**
(45) Date of publication and mention of the grant of the patent: **01.08.2018**
(21) Application number: 11737169.0
(22) Date of filing: 28.01.2011
(51) Int. Cl.: G01N 33/68, G01N 33/53

(54) **METHOD OF ANALYZING HUMAN sCD14-ST**
ANALYSE-VERFAHREN FÜR MENSCHLICHES sCD14-ST
PROCÉDÉ DE DOSAGE DU sCD14-ST HUMAIN

(30) Priority: 29.01.2010 JP 2010018340
(43) Date of publication of application: 05.12.2012
(73) Proprietor: LSI Medience Corporation, Tokyo 101-8517 (JP); MOCHIDA PHARMACEUTICAL CO., LTD., Shinjuku-ku Tokyo 160-8515 (JP)
(72) Inventor: OKAMURA, Yoshikazu, Tokyo 101-8517 (JP); YOKOI, Hiroyuki, Tokyo 108-8559 (JP)
(74) Representative: Vossius & Partner Patentanwälte Rechtsanwälte mbB
(86) International application number: PCT/JP2011/051778
(87) International publication number: WO 2011/093459

(56) References cited:
- EP-A1- 1 746 104
- JP-B- 4 040 666
- YOSHIKAZU OKAMURA ET AL: "Development of a point-of-care assay system for measurement of presepsin (sCD14-ST)", CLINICA CHIMICA ACTA, ELSEVIER BV, AMSTERDAM, NL, vol. 412, no. 23, 26 July 2011 (2011-07-26), pages 2157-2161, XP028309183, ISSN: 0009-8981, DOI: 10.1016/J.CCA.2011.07.024 [retrieved on 2011-08-03]
- HIDEAKI SHIRAI ET AL.: 'Tokutei Kenko Shinsa ni Okeru Kentai no Toriatsukai Part 2: Kentai Saishu kara Enshin Bunri made no Hochi Jikan' JAPANESE JOURNAL OF CLINICAL LABORATORY AUTOMATION vol. 39, no. 4, 01 September 2007, page 426, XP008161408
- YASUSHI TAKAGI: 'Koredake wa Shitte Okitai Kensa no Point (sixth series) Rinsho Kensa Soron Kentai Saishu Teishutsu-ji no Chuiten' MEDICINA vol. 36, no. 11, 30 October 1999, pages 16 - 19, XP008161416
- SHUN'ICHI SASAKI: 'Nichijo Kensa kara Mananda Koto -PT.APTT o Tsujite' THE MEDICAL JOURNAL OF YURI KUMIAI GENERAL HOSPITAL vol. 18, 01 March 2006, pages 285 - 288, XP008161414
- SHIGEATSU ENDO ET AL.: 'Haiketsusho Shindan ni Okeru sCD14-ST no Yuyosei to Kan'i Shindan Kit no Kento' JOURNAL OF JAPANESE ASSOCIATION FOR ACUTE MEDICINE vol. 17, no. 8, 15 August 2006, page 530, XP008161827
- SHIGEATSU ENDO ET AL.: 'Usefulness of Soluble CD 14 Subtype Which as Is a New Diagnostic Marker for Sepsis' JAPAN JOURNAL OF CRITICAL CARE FOR ENDOTOXEMIA vol. 9, no. 1, 09 December 2005, pages 46 - 50, XP008147642
- TATSUYORI SHOZUSHIMA ET AL.: 'Zenshinsei Ensho Hanno Shokogun ni Okeru Haiketsusho Shindan Oyobi Jushodo no Shihyo to shite no sCD14-ST- chi Sokutei no Yuyosei' JOURNAL OF JAPANESE ASSOCIATION FOR ACUTE MEDICINE vol. 21, no. 8, 15 August 2010, page 485, XP008161436

## Description

### TECHNICAL FIELD

The present invention relates to a method of analyzing human sCD14-ST, which is known as a diagnostic marker of sepsis.

The term "analysis" as used herein includes a detection to judge the presence or absence of human sCD14-ST as a substance to be analyzed, and a measurement to quantitatively or semi-quantitatively determine the amount of the substance to be analyzed.

### BACKGROUND ART

The CD14 molecule was named as a protein identified by a family of antibodies which recognize a glycoprotein expressed on the membrane surface of monocytes at the Third Leukocyte Typing Conference in 1986. In 1990, Wright et al. elucidated that the CD14 molecule was a receptor for an endotoxin, LPS (non-patent literature 1). The CD14 molecule is a glycoprotein having a molecular weight of 53 to 55 kDa, and analyses on cDNA revealed that 1.4kb mRNA has a coding sequence of 356 amino acides (non-patent literature 2).

Human CD14 molecules include soluble CD14 as well as membrane-bound CD14, and it is known that a plurality of soluble CD14 subtypes having different molecular weights is present in blood. As these soluble CD14 subtypes, two soluble CD14 of about 55 kDa and 49 kDa (soluble high molecular weight cD14) and a soluble low molecular weight CD14 subtype of about 13 kDa (hereinafter referred to as human sCD14-ST) are known, and human sCD14-ST is clinically useful as a diagnostic marker of sepsis (patent literature 1)

Regarding human sCD14-ST, patent literature 1 discloses that when serum samples are repeatedly frozen and thawed, or allowed to stand at room temperature for a long time, it is assumed that human sCD14-ST in the serum samples is decomposed and cannot be measured in some cases, or it is assumed that high molecular weight CD14 in the serum samples is decomposed to human sCD14-ST or its similar structures and leads to incorrect measured values (paragraph [0151]). Patent literature 1 also discloses that human sCD14-ST is generated by digesting the full length of CD14 with proteases such as elastase, and that the generating sCD14-ST is stably present in the living body (in serum) at least in detectable quantities (paragraph [0126]).

As described above, the cases where incorrect measured values are obtained when serum samples are frozen and thawed, or allowed to stand at room temperature for a long time are assumed in patent literature 1, but it is general information about storage conditions of specimens, and does not caution any special handling. Patent literature 1 rather discloses that human sCD14-ST is stable in the living body and in serum.

### CITATION LIST

### NON-PATENT LITERATURE

[Non-patent literature 1] Science (the United States), 1990, vol. 249, p. 1431-1433
[Non-patent literature 2] Nucleic Acids Research (the United Kingdom), 1988, vol. 16, p. 4173

### PATENT LITERATURE

[Patent literature 1] Japanese Patent No. 4,040,666

### SUMMARY OF INVENTION

### TECHNICAL PROBLEM

Under the circumstances where it has been known that human sCD14-ST is relatively stably present in serum, the present inventors have conducted intensive studies to develop a more accurate method of analyzing human sCD14-ST contained in samples including plasma samples and whole blood samples, and as a result, unexpectedly found that the measured values of human sCD14-ST were affected when whole blood samples were used.

The present invention has been made based on this finding. An object of the present invention is to provide an analysis method capable of accurately measuring human sCD14-ST, even when a whole blood sample is used.

### SOLUTION TO PROBLEM

The problem may be solved by the present invention, that is, a method of analyzing human sCD14-ST, characterized by analyzing human sCD14-ST in a whole blood sample within 6 hours of the collection of the sample.

The term "human sCD14-ST" (also referred to as Presepsin (registered trademark)) as used herein means the "soluble CD14 antigen of the first aspect" disclosed in Japanese Patent No. 4,040,666, and more particularly, is a soluble CD14 antigen having the following characteristics:
1) Molecular weight of 13 ± 2 kDa, as measured by SDS-PAGE under nonreducing conditions;
2) Having the amino acid sequence of SEQ ID NO: 1 at the N-terminal sequence; and
3) Binding specifically to an antibody prepared using a peptide consisting of 16 amino acid residues of SEQ ID NO: 2 as the antigen.

SEQ ID NO: 1 :
SEQ ID NO: 2 :

### ADVANTAGEOUS EFFECTS OF INVENTION

According to the present invention, human sCD14-ST, which is stable in serum or plasma samples, but measured values of which is fluctuating with the progress of time in whole blood samples, can be accurately analyzed using a whole blood sample, without preparing a serum or plasma sample from the whole blood sample.

### BRIEF DESCRIPTION OF THE DRAWINGS

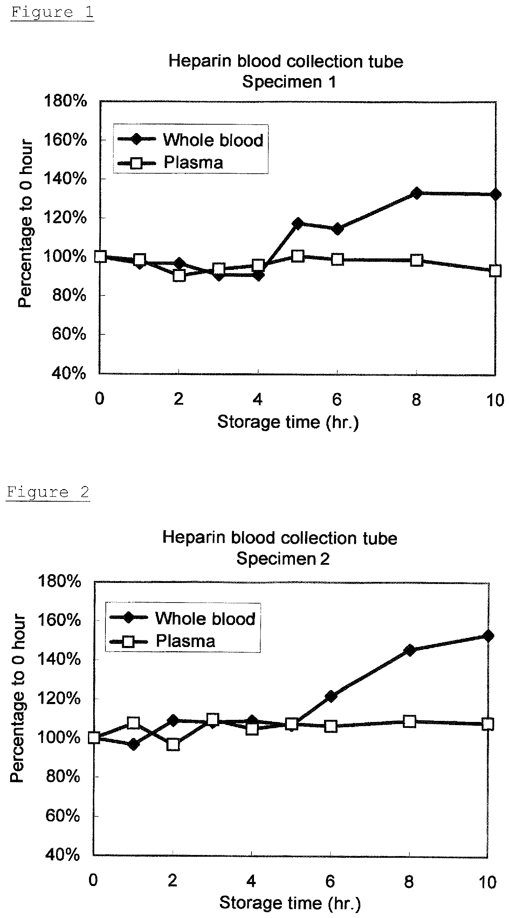

FIG. 1 is a graph showing the results of the measurement of human sCD14-ST in whole blood samples and plasma samples (specimen 1) which were prepared using heparin blood collection tubes and were allowed to stand at room temperature for predetermined periods of time (0, 1 hour, 2 hours, 3 hours, 4 hours, 5 hours, 6 hours, 8 hours, and 10 hours).
FIG. 2 is a graph showing the results of the measurement of human sCD14-ST in whole blood samples and plasma samples (specimen 2) which were prepared using heparin blood collection tubes and were allowed to stand at room temperature for predetermined periods of time (0, 1 hour, 2 hours, 3 hours, 4 hours, 5 hours, 6 hours, 8 hours, and 10 hours).

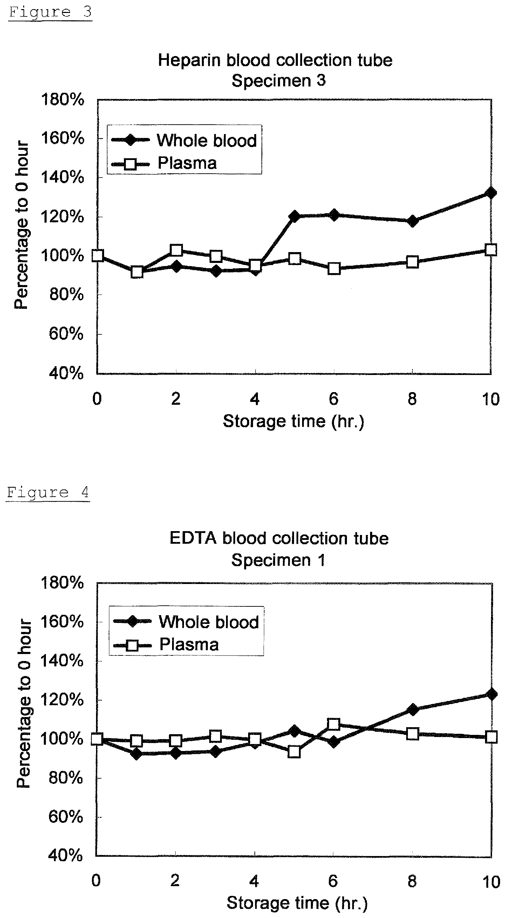

FIG. 3 is a graph showing the results of the measurement of human sCD14-ST in whole blood samples and plasma samples (specimen 3) which were prepared using heparin blood collection tubes and were allowed to stand at room temperature for predetermined periods of time (0, 1 hour, 2 hours, 3 hours, 4 hours, 5 hours, 6 hours, 8 hours, and 10 hours).
FIG. 4 is a graph showing the results of the measurement of human sCD14-ST in whole blood samples and plasma samples (specimen 1) which were prepared using EDTA blood collection tubes and were allowed to stand at room temperature for predetermined periods of time (0, 1 hour, 2 hours, 3 hours, 4 hours, 5 hours, 6 hours, 8 hours, and 10 hours).

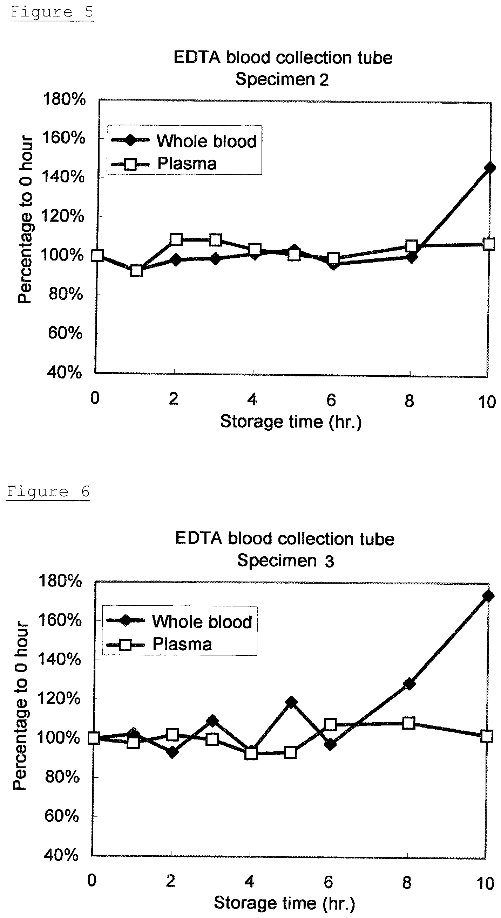

FIG. 5 is a graph showing the results of the measurement of human sCD14-ST in whole blood samples and plasma samples (specimen 2) which were prepared using EDTA blood collection tubes and were allowed to stand at room temperature for predetermined periods of time (0, 1 hour, 2 hours, 3 hours, 4 hours, 5 hours, 6 hours, 8 hours, and 10 hours).
FIG. 6 is a graph showing the results of the measurement of human sCD14-ST in whole blood samples and plasma samples (specimen 3) which were prepared using EDTA blood collection tubes and were allowed to stand at room temperature for predetermined periods of time (0, 1 hour, 2 hours, 3 hours, 4 hours, 5 hours, 6 hours, 8 hours, and 10 hours).

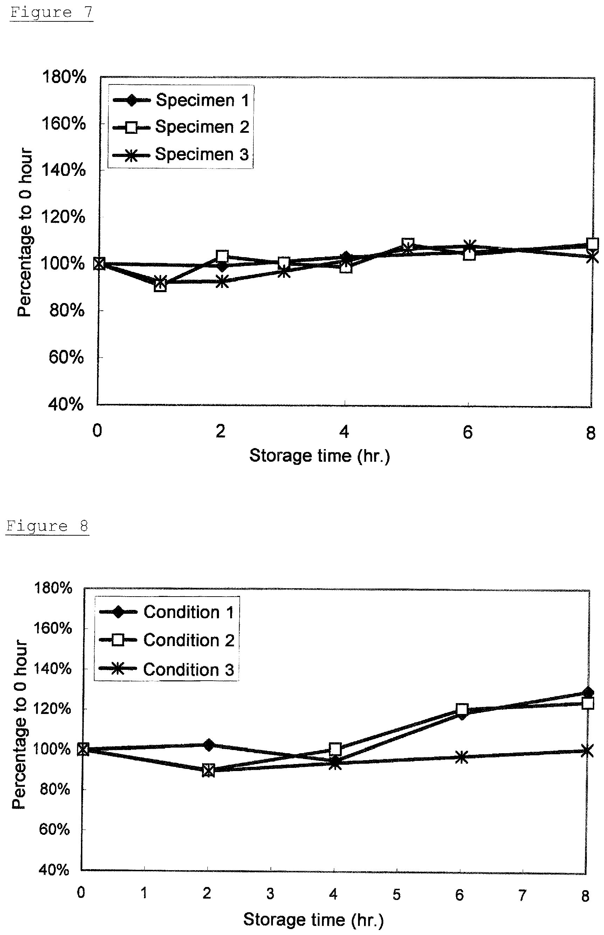

FIG. 7 is a graph showing the results of the measurement of human sCD14-ST in serum samples (specimens 1 to 3) which were allowed to stand at room temperature for predetermined periods of time (0, 1 hour, 2 hours , 3 hours, 4 hours, 5 hours, 6 hours, and 8 hours).
FIG. 8 is a graph showing the results of the measurement of human sCD14-ST in samples which were allowed to stand under various conditions (conditions 1 to 3), in order to examine the factors of instability of whole blood samples.

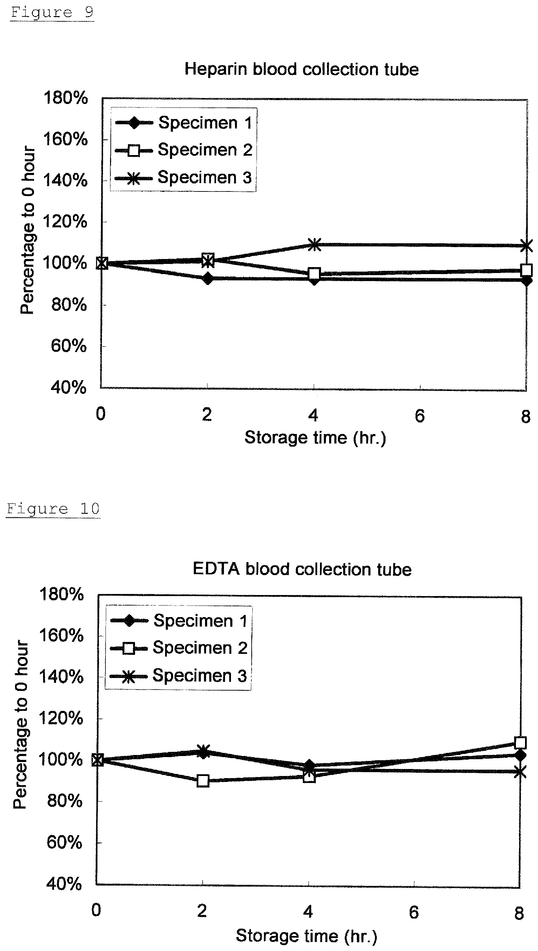

FIG. 9 is a graph showing the results of the measurement of CRP in whole blood samples (specimens 1 to 3) which were prepared using heparin blood collection tubes and were allowed to stand at room temperature for predetermined periods of time (0, 2 hours, 4 hours, and 8 hours).
FIG. 10 is a graph showing the results of the measurement of CRP in whole blood samples (specimens 1 to 3) which were prepared using EDTA blood collection tubes and were allowed to stand at room temperature for predetermined periods of time (0, 2 hours, 4 hours, and 8 hours).

### DESCRIPTION OF EMBODIMENTS

The analysis method of the present invention may be carried out in accordance with known analysis methods for human sCD14-ST, except that a whole blood sample is used as the sample to be analyzed, and that its analysis is carried out within 6 hours of the collection of the sample.

Human sCD14-ST may be analyzed using various known analysis methods for proteins, for example, immunological analysis methods using one or more antibodies, or biochemical methods such as electrophoresis, and an automatic analyzer for clinical testing may be used.

For example, Japanese Patent No. 4,040,666 discloses a method of measuring human sCD14-ST, more particular, a sandwich EIA system [Example 7-(1) of Japanese Patent No. 4,040,666] using the combination of a polyclonal antibody (S68 antibody) or a monoclonal antibody (F1146-17-2 antibody) prepared using a peptide (S68 peptide described in Japanese Patent No. 4,040,666) consisting of 16 amino acid residues of SEQ ID NO: 2 as the antigen, and an anti-CD14-antigen monoclonal antibody (for example, F1031-8-3 antibody or F1106-13-3 antibody), and it may be applied to the analysis method of the present invention.

In the analysis method used in the Examples described below, human sC014-ST contained in each sample is bound to magnetic latex coated with an anti-sCD14-ST mouse monoclonal antibody (F1106-13-3 antibody) and an ALP (alkaline phosphatase)-labeled anti-sCD14-ST rabbit polyclonal antibody (S68 antibody) to form an immunocomplex by an antigen-antibody reaction; the excess ALP-labeled anti-sCD14-ST rabbit polyclonal antibody is removed from the immunocomplex; and the ALP enzyme activity of the immunocomplex is measured by a chemiluminescent enzyme immunoassay (CLEIA) using a luminescent substrate (CDP-Star; Tropix) to determine the concentration of human sCD14-ST contained in the sample.

In the analysis method of the present invention, a whole blood sample is used as the sample to be analysis. The whole blood sample may be collected by a normal operation, i.e., using a blood collection tube containing an anticoagulant such as heparin, EDTA, or citric acid.

The whole blood sample collected using a blood collection tube may be stirred by inversion several times. According to the findings of the present inventors including the Examples below, when human sCD14-ST contained in a whole blood sample is measured, the measured values may fluctuate with the progress of time in some cases, and excessive physical shock when preparing the sample or transporting the sample may be a factor which affects the measured values in some cases.

In the analysis method of the present invention, the analysis is carried out within 6 hours (preferably within 4 hours) of the collection of the sample. The expression "the analysis is carried out within 6 hours" as used herein means that the analysis begins within 6 hours.

More particularly, it is preferable that when the whole blood sample is collected using an EDTA blood collection tube, the analysis is carried out within 6 hours, and it is preferable that when the whole blood sample is collected using a heparin blood collection tube, the analysis is carried out within 4 hours.

As shown in the concrete experimental data described in the Examples below, an increase in the measured values of human sCD14-ST was observed in whole blood samples from healthy people, even when the measurement was after 6 to 8 hours from the blood collection, in which period no changes in measured values are observed in plasma samples or serum samples. It is known that the measured value of human sCD14-ST is different by 50-fold between healthy people and sepsis patients, and the concentration of human sCD14-ST contained in the blood of healthy people is extremely low. This enables sepsis to be diagnosed with high sensitivity and high specificity by setting an appropriate cut-off value (Example 12 of patent literature 1). However, where the measured values of human sCD14-ST in healthy people increase with the progression of time after collecting a whole blood sample, there is a possibility that the judgment of whether the measured value is lower or higher than the cut-off value is different depending on the measuring time. Under these circumstances, the present inventors found that the measured values of human sCD14-ST in a whole blood sample did not fluctuate within 6 hours (preferably within 4 hours) of the collection of a sample. Since the analysis method of the present invention is carried out within 6 hours of the collection of a whole blood sample, human sCD14-ST can be accurately measured without an increase in measured values.

### EXAMPLES

The present invention now will be further illustrated by, but is by no means limited to, the following Examples.

### <<EXAMPLE 1: Method of measuring human sCD14-ST in samples>>

Each sample to measure human sCD14-ST was measured using reagents for measuring human sCD14-ST. These reagents included a solution of magnetic latex coated with an anti-sCD14-ST mouse monoclonal antibody (F1106-13-3 antibody; Japanese Patent No. 4,040,666) as the first antibody solution, a solution of an ALP (alkaline phosphatase)-labeled anti-sCD14-ST rabbit polyclonal antibody (S68 antibody) as the second antibody solution, a washing solution for B/F separation, a substrate solution, and the like, and were packaged in a cartridge applicable to an automatic luminescent immunoanalyzer described below.

An automatic luminescent immunoanalyzer (PATHFAST; Mitsubishi chemical Medience Corporation), which is similar to that disclosed in Japanese Patent No. 3,115,501 and which can automatically perform immunoassay using magnetic particles, was used for the measurement. This apparatus is capable of efficiently performing B/F separation by magnetic force in a tip arranged as a unit of liquid suction and discharge, and exhibits high efficiency in washing. The measurement steps of the apparatus are as follows.

A chemiluminescent substrate "CDP-Star" (Tropix) was used as the substrate, and emission counts detected by a photomultiplier tube (PMT) were regarded as the measurement results.

Each cartridge for an automatic measurement was filled with each sample, a sample diluent solution, the solution of magnetic particles (coated with the first antibody), the washing liquid for B/F separation, the solution of the second antibody, the substrate solution, and the like, and loaded to the automated analyzer. The following steps were carried out in accordance with the normal procedure:
(1) The sample solution previously adjusted to predetermined dilution ratio with a sample diluent solution, the solution of magnetic particles, and the solution of the second antibody were mixed to generate an immunocomplex by an antigen-antibody reaction.
(2) A B/F separation was carried out to remove unreacted substances as follows. The resulting reaction solution was aspirated into the tip arranged as a unit for aspirating a solution, and the magnetic particles were trapped by contact with a magnet on the outer wall of the tip. The solution was discharged from the tip while the magnetic particles were trapped on the inner wall of the tip. After separation, the washing liquid for the B/F separation held in another reaction vessel was aspirated and discharged to wash the magnetic particles in the tip.
(3) The magnet was removed from the outer wall of the tip. The substrate solution was aspirated and discharged to disperse the magnetic particles trapped on the inner wall of the tip and carry out an enzyme reaction.
(4) The amount of luminescence was measured by PMT.

### <<EXAMPLE 2: Examination of measured values in whole blood, plasma and serum samples after short-term storage>>

Whole blood samples, serum samples, and plasma samples were allowed to stand at room temperature for predetermined periods of time, and then used to measure human sCD14-ST in accordance with the measuring method described in Example 1.

More particularly, whole blood samples, serum samples, and plasma samples, which had been collected from three healthy people using blood collection tubes containing an anticoagulant (heparin or EDTA), were allowed to stand at room temperature for 0, 1 hour, 2 hours, 3 hours, 4 hours, 5 hours, 6 hours, 8 hours, or 10 hours (only for the whole blood samples and the plasma samples), and each measurement was immediately carried out at each time point to confirm the time course of the measured values for each sample. As the serum samples, whole blood samples were collected using plain blood collection tubes (Terumo) and centrifuged and separated into sera, and these serum samples were used. EDTA plasma samples were prepared using EDTA blood collection tubes (Terumo), and heparin plasma samples were prepared using heparin blood collection tubes (Terumo). The whole blood samples were collected using the above blood collection tubes, and directly used without plasma separation.

The results of the whole blood samples and the plasma samples prepared using heparin blood collection tubes are shown in Figures 1 to 3 (specimens 1 to 3), and the results of the whole blood samples and the plasma samples prepared using EDTA blood collection tubes are shown in Figures 4 to 6 (specimens 4 to 6). When the value measured after the samples were collected and immediately treated is regard as 100%, each increase or decrease from the initial value is indicated as percentage in Figures 1 to 6. The values within the range of 90% to 110% are evaluated as "stable". The measured values in the whole blood samples tended to increase after 5 to 8 hours from the blood collection, but no changes in the measured values were observed in the plasma samples which had been allowed to stand under the same conditions. The measured values in the whole blood samples prepared using EDTA blood collection tubes were stable for 6 hours, and the measured values in the whole blood samples prepared using heparin blood collection tubes were stable for 4 hours.

In addition, when excessive physical shock, such as stirring, was applied to the whole blood samples, a remarkable increase in the measured values was observed (data not shown).

The results of the serum samples are shown in Figure 7. No changes were detected in the serum samples which had been allowed to stand under the same conditions.

Further, it was confirmed that the measured values were sufficiently stable in the serum samples and the plasma samples, even when they had been allowed to stand at room temperature for at least 24 hours (data not shown).

### <<EXAMPLE 3: Examination of stability of whole blood samples>>

The cause of the instability of the measured values of the whole blood samples was examined.
Condition 1: Whole blood sample
Condition 2: Plasma sample prepared by allowing the whole blood sample to stand at room temperature for predetermined periods of time and performing plasma separation before the measurement
Condition 3: Plasma sample

The preparation and the measurement of the samples were carried out, using heparin blood collection tubes, in accordance with Example 2. The results are shown in Figure 8. The measured values in the whole blood samples (Condition 1) increased after 4 or more hours from the blood collection, as shown in Example 1, whereas the measured values in the plasma samples (Condition 3) were stable for 8 hours. In contrast, an increase in the measured values was observed in the plasma samples of Condition 2, which had been separated from the whole blood samples stored for predetermined periods of time, as similar to the whole blood samples (Condition 1).

It was suggested from these results that the increase in the measured values was caused by factor(s) not from the plasma component but from the blood cell component. It was necessary to measure a whole blood sample directly within 4 hours of the blood collection, and further, unless plasma separation was carried out within 4 hours of the blood collection of a whole blood sample, an increase in the measured values after the plasma separation was observed, and therefore, it was found that the plasma separation within 4 hours of the blood collection was necessary to obtain accurate measured values.

### <<Referential Example 1>>

Whole blood samples were collected from three healthy people using blood collection tubes containing an anticoagulant (heparin or EDTA), and were allowed to stand at room temperature for 0, 2 hours, 4 hours, and 8 hours, and each measurement was immediately carried out at each time point, using a commercially available CRP measuring reagent (PATHFAST hs CRP measuring reagent; Mitsubishi chemical Medience Corporation) to confirm the time course of the measured values for each sample. The preparation and the measurement of the samples were carried out in accordance with Example 2, except that the CRP measuring reagent was used.

The results are shown in Figure 9 (heparin blood collection tube) and Figure 10 (EDTA blood collection tube). Unlike the case of human sCD14-ST in Example 2, the measured values did not tend to increase after 4 or more hours from the blood collection, and it was confirmed that CRP was stable in whole blood samples.

### INDUSTRIAL APPLICABILITY

The analysis method of the present invention may be used in, for example, the diagnosis of sepsis.

## Claims

1. A method of analyzing human sCD14-ST, **characterized by** analyzing human sCD14-ST in a whole blood sample without plasma separation within 6 hours of the collection of the sample.

2. The method according to claim 1, wherein human sCD14-ST in the whole blood sample is analyzed within 4 hours of the collection of the sample.

3. The method according to claim 1 or 2, wherein the analysis is carried out using an untreated whole blood sample.

4. The method according to any one of claims 1 to 3, wherein the whole blood sample is collected using a heparin blood collection tube or an EDTA blood collection tube.

## Patentansprüche

1. Verfahren zum Analysieren von menschlichem sCD14-ST, **gekennzeichnet durch** Analysieren von menschlichem sCD14-ST in einer Vollblutprobe ohne Abtrennung von Plasma, innerhalb von 6 Stunden nach Einsammeln der Probe.

2. Verfahren nach Anspruch 1, wobei menschliches sCD14-ST in der Vollblutprobe innerhalb von 4 Stunden nach Einsammeln der Probe analysiert wird.

3. Verfahren nach Anspruch 1 oder 2, wobei die Analyse unter Verwendung einer unbehandelten Vollblutprobe durchgeführt wird.

4. Verfahren nach einem der Ansprüche 1 bis 3, wobei die Vollblutprobe mittels eines Heparin-Blutsammelröhrchens oder eines EDTA-Blutsammelröhrchens eingesammelt wird.

## Revendications

1. Procédé de dosage du sCD14-ST humain, **caractérisé en ce que** le dosage du sCD14-ST humain dans un échantillon de sang total sans séparation du plasma est réalisé dans les 6 heures suivant le prélèvement de l'échantillon.

2. Procédé selon la revendication 1, dans lequel le sCD14-ST humain dans l'échantillon de sang total est dosé dans les 4 heures suivant le prélèvement de l'échantillon.

3. Procédé selon la revendication 1 ou 2, dans lequel le dosage est effectué en utilisant un échantillon de sang total non traité.

4. Procédé selon l'une quelconque des revendications 1 à 3, dans lequel l'échantillon de sang total est prélevé en utilisant un tube de prélèvement de sang contenant de l'héparine ou un tube de prélèvement de sang contenant de l'EDTA.


### SEQUENCE LISTING

<110> Mitsubishi Chemical Medience Corporation Mochida Pharmaceutical Co., Ltd.
<120> Method of analyzing human sCD14-ST
<130> MCM-870
<150> JP 2010-018340
   <151> 2010-01-29
<160> 2
<170> PatentIn version 3.5
<210> 1
   <211> 11
   <212> PRT
   <213> Homo sapiens
<400> 1
   Thr Thr Pro Glu Pro Cys Glu Leu Asp Asp Glu
   1               5                   10
<210> 2
   <211> 16
   <212> PRT
   <213> Homo sapiens
<400> 2
   Arg Val Asp Ala Asp Ala Asp Pro Arg Gln Tyr Ala Asp Thr Val Lys
   1               5                   10                  15